# FASCICULE DE BREVET EUROPEEN

(11) **EP 4 452 227 B1**
(45) Date de publication et mention de la délivrance du brevet: **26.11.2025**
(21) Numéro de dépôt: 22847581.0
(22) Date de dépôt: 19.12.2022
(51) Int. Cl.: A61K 9/00, A61K 47/10, A61K 47/24

(54) **COMPOSITION PHARMACEUTIQUE COMPRENANT DU SALBUTAMOL**
PHARMAZEUTISCHE ZUSAMMENSETZUNG MIT SALBUTAMOL
PHARMACEUTICAL COMPOSITION COMPRISING SALBUTAMOL

(30) Priorité: 20.12.2021 FR 2113999
(43) Date de publication de la demande: 30.10.2024
(73) Titulaire: Aptar France SAS, 27110 Le Neubourg (FR)
(72) Inventeur: PEYRON, Isabelle, 81100 Castres (FR); ROSSI, Irene, 63074 San Benedetto del Tronto (Ascoli Piceno) (IT); SARRAILH, Ségolène, 27400 Heudebouville (FR)
(74) Mandataire: Santarelli
(86) Numéro de dépôt international: PCT/FR2022/052421
(87) Numéro de publication internationale: WO 2023/118717

(56) Documents cités:
- US-A1- 2007 041 911
- US-A1- 2021 244 688

## Description

### Domaine technique

La présente invention se rapporte à une composition pharmaceutique qui est susceptible d'être utilisée dans le traitement de troubles respiratoires et qui comprend un principe actif à base d'un sel de salbutamol, en particulier du sulfate de salbutamol, et un gaz propulseur formé par du 1,1-difluoroéthane.

L'invention se rapporte également à une cartouche comprenant cette composition pharmaceutique ainsi qu'à un aérosol-doseur muni d'une telle cartouche.

L'invention se rapporte enfin aux utilisations de cette composition pharmaceutique et de cette cartouche dans un aérosol-doseur.

### État de la technique antérieure

Le salbutamol ainsi que ses dérivés sont des principes actifs connus comme bronchodilatateurs dans le traitement de troubles respiratoires tels que l'asthme et les broncho-pneumopathies chroniques obstructives (BPCO).

Les compositions pharmaceutiques comprenant du salbutamol ou l'un de ses dérivés sont classiquement délivrées aux patients au moyen d'un aérosol-doseur (en anglais "metered dose inhaler" ou MDI).

Un aérosol-doseur est un dispositif d'administration équipé d'une cartouche contenant la composition pharmaceutique, d'une valve doseuse permettant de distribuer une quantité contrôlée de composition pharmaceutique contenant le principe actif et d'un applicateur permettant d'exercer une pression sur la valve doseuse et muni d'un embout buccal.

La composition pharmaceutique comprend un gaz propulseur dans lequel le principe actif est dissous, suspendu ou dispersé, et éventuellement un ou plusieurs autres composés qui peuvent être notamment choisis parmi des tensioactifs, des excipients polaires et des conservateurs.

Le choix du gaz propulseur mis en œuvre dans les aérosols-doseurs à finalité pharmaceutique a évolué au cours des années.

Compte tenu de leurs effets délétères sur la couche d'ozone, les chlorofluorocarbones (CFC), longtemps utilisés, ont été abandonnés au profit d'hydrofluorocarbures (HFC), plus récemment désignés par le terme d'hydrofluoroalcanes (HFA), tels que le 1,1,1,2-tétrafluoroéthane (HFC-134a, HFA-134a ou R-134a) et le 1,1,1,2,3,3,3-heptafluoropropane (HFC-227ea, HFA-227ea ou R-227ea) qui sont des composés ne présentant pas d'effet néfaste sur la couche d'ozone ni de toxicité humaine.

Toutefois, ces hydrofluoroalcanes R-134a et R-227ea se caractérisant par un fort potentiel de réchauffement global (PRG) avec un impact non négligeable sur l'effet de serre, des compositions pharmaceutiques comprenant du salbutamol, ou l'un de ses dérivés, et un gaz propulseur alternatif ont été proposées.

Ainsi, les documents WO 2013/054137 A1, WO 2014/170689 A1 et WO 2013/054135 A1, respectivement référencés [1] à [3] dans la suite de la présente description, décrivent la mise en œuvre d'un hydrofluorocarbure (HFC) ou hydrofluoroalcane (HFA) particulier, le 1,1-difluoroéthane (HFC-152a, HFA-152a ou R-152a) dans des compositions pharmaceutiques comprenant du sulfate de salbutamol.

Le document US 2007/041911 A1, référencé [4], cite incidemment le HFC-152a parmi un certain nombre d'hydrofluorocarbures susceptibles d'être utilisés comme gaz propulseur dans des compositions pharmaceutiques comprenant un sel d'addition acide de salbutamol, un co-solvant ainsi qu'un acide organique ou inorganique. Toutefois, le document [4] ne décrit aucun exemple de composition pharmaceutique mettant spécifiquement en œuvre le R-152a.

Le récent document US 2021/244688 A1, référencé [5], décrit des compositions pharmaceutiques comprenant du salbutamol, seul ou en association avec au moins un antagoniste muscarinique à action prolongée et/ou au moins un corticostéroïde, ainsi que du HFA-152a comme gaz propulseur. Or, dans le document [5], le salbutamol mis en œuvre est un salbutamol dit "base", qui est défini comme excluant tous les dérivés pharmaceutiquement acceptables du salbutamol, en particulier les sels de salbutamol.

Plus particulièrement, les compositions pharmaceutiques décrites dans les documents [1] et [2] comprennent du sulfate de salbutamol, du R-152a ainsi qu'un ou plusieurs tensioactifs dont le rôle est d'aider à la dispersion des particules de principe actif dans le gaz propulseur.

Dans le document [1], ce tensioactif est l'acide oléique alors que, dans le document [2], ce tensioactif comprend au moins un composé autre que l'acide oléique.

Bien qu'il ne s'agisse pas de variantes particulièrement privilégiées, les compositions pharmaceutiques décrites dans les documents [1] et [2] peuvent également comprendre un ou plusieurs excipients polaires, tels que l'éthanol, qui sont décrits comme ayant pour effet de solubiliser le tensioactif dans le propulseur et/ou d'inhiber le dépôt de particules de principe actif sur les surfaces de la cartouche.

*A contrario,* les compositions pharmaceutiques décrites dans le document [3] ne comprennent pas de tensioactif au motif que les tensioactifs ne seraient pas souhaitables et qu'il y aurait un intérêt à former une suspension stable sans l'utilisation de tensioactif. Le document [3] précise que l'utilisation du gaz propulseur R-152a permet de préparer des compositions pharmaceutiques exemptes de tensioactifs et d'excipients polaires qui présentent cependant de bonnes performances pharmaceutiques lorsqu'elles sont délivrées à partir d'un dispositif d'administration de médicaments tel qu'un aérosol-doseur (MDI).

Or, les Inventeurs ont constaté que des compositions pharmaceutiques constituées uniquement de sulfate de salbutamol et de R-152a ne permettaient pas d'obtenir les performances d'aérosolisation escomptées.

C'est donc sur la base de ce constat et dans un souci constant d'amélioration des propriétés d'aérosolisation et, partant, des propriétés thérapeutiques conférées par les compositions pharmaceutiques destinées au traitement de troubles respiratoires qu'est basée la présente invention.

### Exposé de l'invention

Ce but ainsi que d'autres sont atteints, en premier lieu, par une composition pharmaceutique du type précité, c'est-à-dire qui comprend un principe actif à base de salbutamol et de 1,1-difluoroéthane en tant que gaz propulseur.

Selon l'invention, la composition pharmaceutique est constituée des composés suivants :
(a) du sulfate de salbutamol en tant que principe actif,
(b) du 1,1-difluoroéthane (R-152a), et
(c) de l'éthanol.

Les Inventeurs ont constaté que, de manière inattendue et surprenante, une composition pharmaceutique qui ne comprend que du sulfate de salbutamol, du R-152a et de l'éthanol permet d'atteindre des performances d'aérosolisation bien supérieures à celles d'une composition pharmaceutique ne comprenant qu'un sel de salbutamol et du R-152a telle que décrite dans le document [3], ces performances d'aérosolisation remarquables étant en outre stables dans le temps.

Ce résultat est d'autant plus inattendu qu'il va à l'encontre des enseignements des documents [1] à [3] qui recommandent de limiter, voire d'éviter la mise en œuvre d'éthanol dans les compositions pharmaceutiques à base de sulfate de salbutamol et de R-152a. Ces documents rapportent notamment que l'éthanol peut provoquer une irritation inacceptable de la bouche et de la gorge, en particulier chez les jeunes patients, et/ou engendrer une pulvérisation grossière des compositions pharmaceutiques se caractérisant par des tailles de gouttelettes trop importantes pour permettre d'obtenir une pénétration acceptable dans les bronchioles profondes du poumon.

Le principe actif (a) se présente avantageusement sous forme de particules dont la taille est adaptée à une délivrance par inhalation de la composition pharmaceutique dans laquelle il est contenu. De manière classique, le diamètre médian des particules de principe actif (a), couramment noté Dv₅₀, est inférieur ou égal à 6 µm, ce qui signifie qu'au moins 50 % en volume des particules de principe actif (a) possèdent un diamètre inférieur ou égal à 6 µm.

Ce diamètre médian des particules de principe actif (a), est avantageusement inférieur ou égal à 5 µm, de préférence compris entre 0,5 µm et 5 µm et, plus préférentiellement, compris entre 1 µm et 4 µm.

Dans une variante de la composition selon l'invention, la proportion massique de principe actif (a) est comprise entre 0,05 % et 0,5 % en masse par rapport à la masse totale de la composition pharmaceutique. Cette proportion massique est avantageusement comprise entre 0,1 % et 0,4 % en masse et, de préférence, comprise entre 0,2 % et 0,35 % en masse par rapport à la masse totale de la composition pharmaceutique.

Dans une variante de la composition selon l'invention, la proportion massique de 1,1-difluoroéthane (b) est comprise entre 89,5 % et 99,9 % en masse par rapport à la masse totale de la composition pharmaceutique.

Dans une autre variante, la proportion massique de 1,1-difluoroéthane (b) est comprise entre 94,5 % et 99,9 % en masse par rapport à la masse totale de la composition pharmaceutique. Cette proportion massique est avantageusement comprise entre 96,6 % et 99,7 % en masse et, de préférence, comprise entre 97,65 % et 99,3 % en masse par rapport à la masse totale de la composition pharmaceutique.

Dans une variante de la composition selon l'invention, la proportion massique d'éthanol (c) est comprise entre 0,05 % et 10 % en masse par rapport à la masse totale de la composition pharmaceutique.

Dans une autre variante, la proportion massique d'éthanol (c) est comprise entre 0,05 % et 5 % en masse par rapport à la masse totale de la composition pharmaceutique.

Les performances d'aérosolisation de la composition pharmaceutique selon l'invention peuvent être atteintes avec une proportion massique d'éthanol relativement faible, qui ne présente aucun danger pour la santé du patient, même jeune.

La proportion massique d'éthanol peut avantageusement être comprise entre 0,2 % et 3 % en masse et, de préférence, comprise entre 0,5 % et 2 % en masse par rapport à la masse totale de la composition pharmaceutique.

La présente description divulgue aussi une composition pharmaceutique pour une utilisation dans le traitement de patients souffrant ou susceptibles de souffrir de troubles respiratoires.

Cette composition pharmaceutique, qui est utilisée dans le traitement de troubles respiratoires, est telle que définie ci-dessus, c'est-à-dire qu'elle est constituée des composés suivants :
(a) (a) du sulfate de salbutamol en tant que principe actif,
(b) du 1,1-difluoroéthane (R-152a), et
(c) de l'éthanol.

Les caractéristiques décrites précédemment en liaison avec la composition pharmaceutique et, notamment, les caractéristiques relatives au principe actif ainsi qu'aux proportions massiques des différents composés formant cette composition pharmaceutique, sont bien entendu applicables à la présente utilisation dans le traitement de troubles respiratoires.

De tels troubles respiratoires peuvent être l'asthme ou encore les broncho-pneumopathies chroniques obstructives (BPCO).

Les patients peuvent être traités par l'administration d'une quantité efficace d'un point de vue thérapeutique d'une composition pharmaceutique telle que définie ci-dessus.

La présente invention se rapporte, en second lieu, à une cartouche comprenant une composition pharmaceutique ainsi qu'à un aérosol-doseur comprenant un telle cartouche.

Selon l'invention, cette composition pharmaceutique est telle que définie ci-dessus, c'est-à-dire qu'elle est constituée du principe actif (a), de R-152a comme gaz propulseur et d'éthanol, les caractéristiques relatives à ces composés pouvant être prises seules ou en combinaison.

La présente invention se rapporte, en troisième lieu, à l'utilisation d'une composition pharmaceutique et/ou d'une cartouche telles que définies ci-dessus dans un aérosol-doseur (MDI), un tel dispositif étant classiquement utilisé pour délivrer des compositions pharmaceutiques comprenant un principe actif à base de salbutamol ou d'un sel pharmaceutiquement acceptable de celui-ci.

D'autres caractéristiques et avantages de l'invention apparaîtront mieux à la lecture du complément de description qui suit, qui se rapporte à des exemples de compositions pharmaceutiques ainsi qu'à l'évaluation de leurs performances d'aérosolisation *in vitro,* deux compositions pharmaceutiques, notées C4 et C4', étant conformes à l'invention, les autres étant des compositions pharmaceutiques comparatives conformes aux enseignements des documents [1] et [3], notées C1 à C3 et C3'.

### Brève description des figures

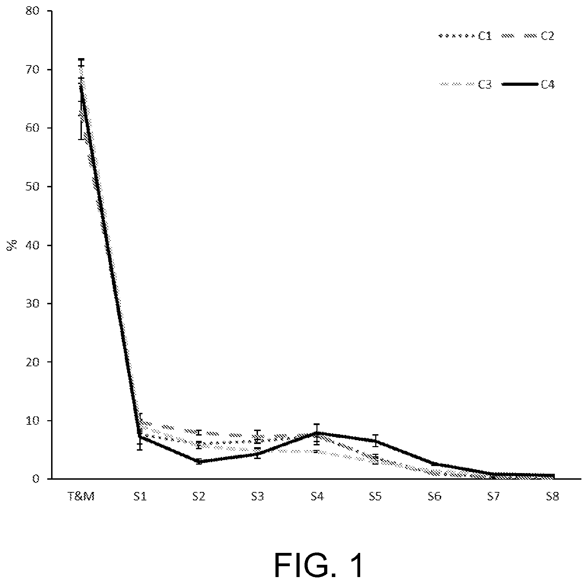

La figure 1 représente les graphes illustrant la fraction déposée de particules de salbutamol (exprimée en %) provenant de doses ou bouffées des compositions pharmaceutiques C1 à C4 telle que mesurée à T0, en fonction des étages de l'impacteur pharmaceutique NGI.

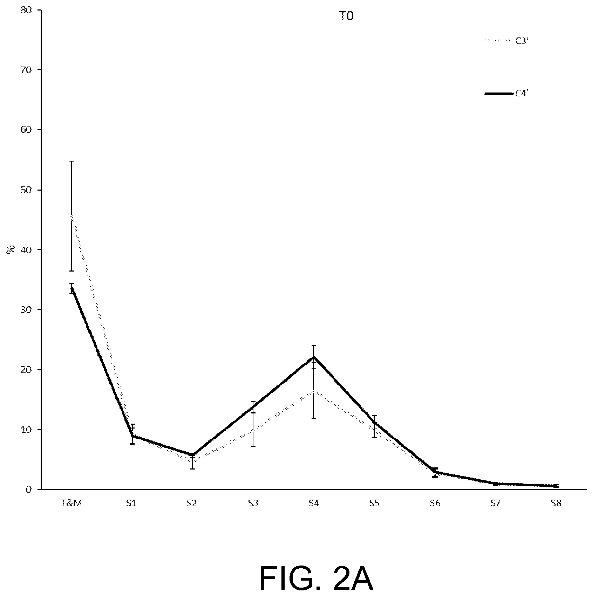

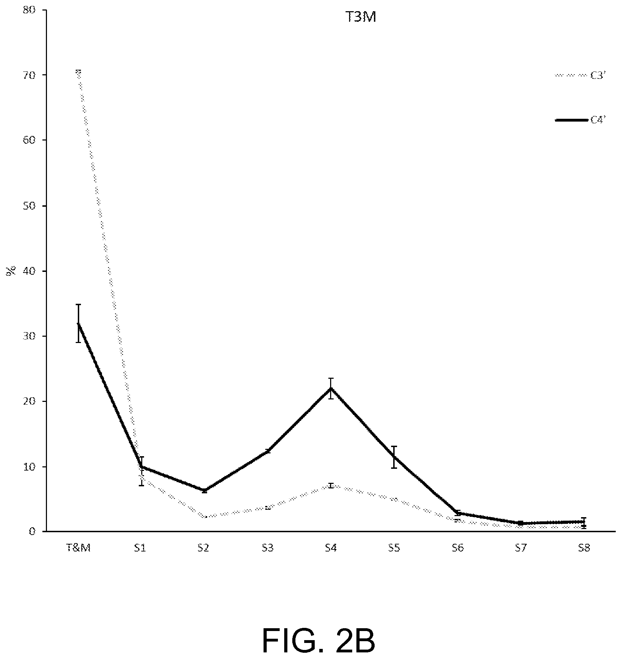

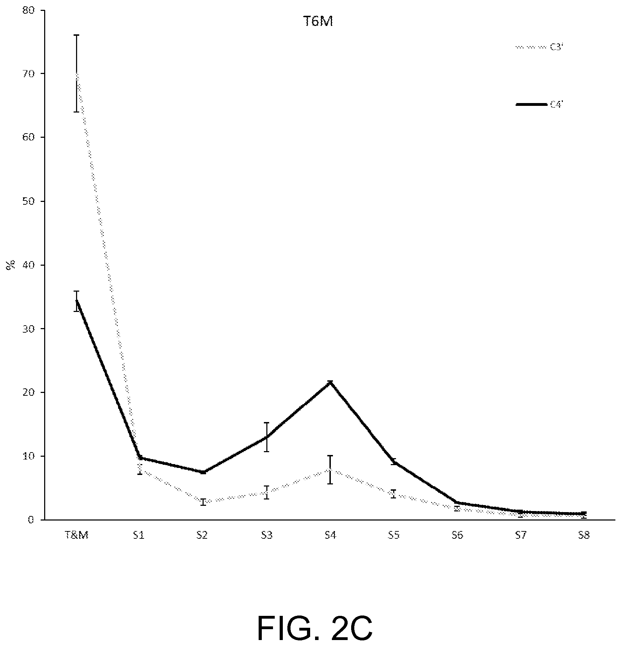

Les figures 2A, 2B et 2C représentent les graphes illustrant la fraction déposée de particules de salbutamol (exprimée en %) provenant de doses ou bouffées des compositions pharmaceutiques C3' et C4' telle que respectivement mesurée à T0, à T3M et à T6M, en fonction des étages de l'impacteur pharmaceutique NGI.

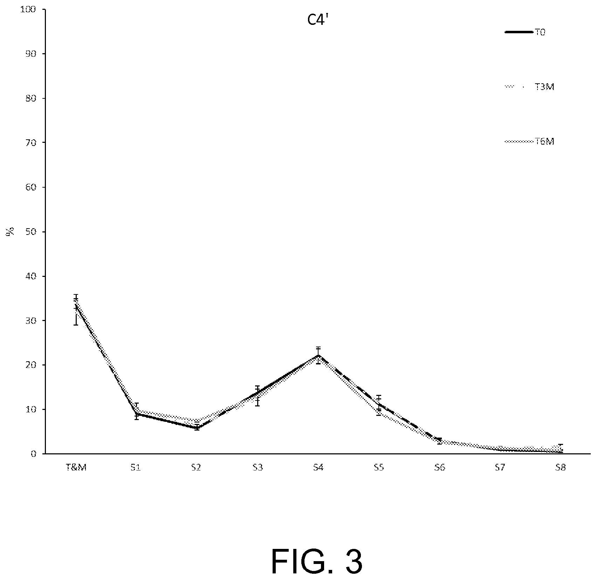

La figure 3 reproduit les graphes des figures 2A, 2B et 2C illustrant la fraction déposée de particules de salbutamol (exprimée en %) provenant des doses de la composition pharmaceutique C4' conforme à l'invention telle que mesurée à T0, à T3M et à T6M, en fonction des étages de l'impacteur pharmaceutique NGI.

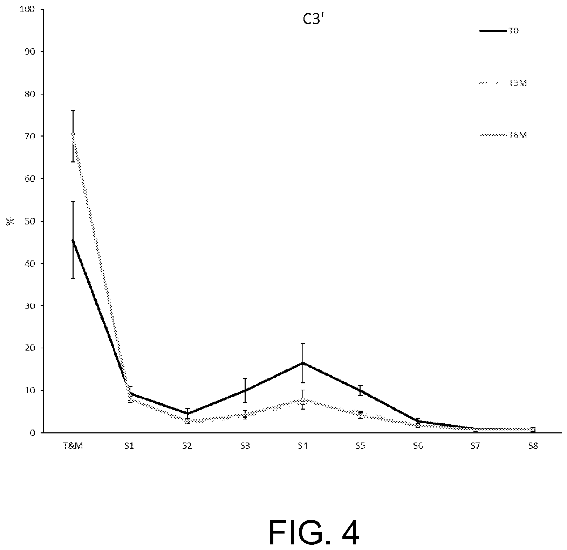

La figure 4 reproduit les graphes des figures 2A, 2B et 2C illustrant la fraction déposée de particules de salbutamol (exprimée en %) provenant des doses de la composition pharmaceutique comparative C3' telle que mesurée à T0, à T3M et à T6M, en fonction des étages de l'impacteur pharmaceutique NGI.

### Exposé détaillé de modes de réalisation particuliers

### Exemple 1

Les aérosols qui ont fait l'objet des tests ont été fabriqués avec les mêmes lots de composés, de cartouches en aluminium et de valves doseuses.

Quatre compositions pharmaceutiques C1 à C4 ont été préparées à partir des quantités suivantes de sulfate de salbutamol (diamètre médian de l'ordre de 2 µm), d'acide oléique et d'éthanol mentionnées dans le tableau 1 ci-dessous.

**Tableau 1**

| Composition | Sulfate de salbutamol (mg) | Acide oléique (mg) | Ethanol (mg) |
|---|---|---|---|
| C1 | 30,125 +/- 2,5 % | 28,438 +/- 2,5 % | 0 |
| C2 | 30,125 +/- 2,5 % | 34,125 +/- 2,5 % | 0 |
| C3 | 30,125 +/- 2,5 % | 0 | 0 |
| C4 | 30,125 +/- 2,5 % | 0 | 113,750 +/- 2,5 % |

Le cas échéant, l'acide oléique ou l'éthanol a tout d'abord été introduit manuellement dans quatre séries distinctes de cartouches suivi du sulfate de salbutamol.

Une valve doseuse a ensuite été sertie sur chacune des cartouches avec un équipement adéquat, puis le gaz propulseur R-152a a été introduit au moyen d'un équipement adapté via la valve doseuse pour atteindre une masse totale de composition pharmaceutique de 11375 mg.

Les aérosols ainsi conditionnés ont alors été stockés en position dite inversée (valve vers le bas) pendant une période de quarantaine d'au moins une semaine.

À l'issue de cette période de quarantaine, un test de mesure de distribution granulométrique aérodynamique (en anglais "aerodynamic particle size distribution" ou APSD) a été réalisé.

Ce test à T0, qui consiste à évaluer la taille aérodynamique des particules de principe actif sortant de la valve, a été réalisé au moyen d'un impacteur pharmaceutique à multi-étages permettant une modélisation approximative de l'arbre bronchique. Dans le cas présent, l'impacteur pharmaceutique utilisé a été le Next Generation Impactor (NGI) qui correspond à l'appareil E de la Pharmacopée Européenne.

Plus particulièrement, les tests ont été réalisés à un débit de 30 L/min, en expulsant 5 doses de chacune des compositions C1 à C4 dans l'impacteur NGI.

Le graphe de la figure 1 représente la fraction de salbutamol déposée dans la gorge et dans la bouche (notée T&M) et sur chacun des étages de l'impacteur (notés S1 à S8).

Pour assurer une efficacité thérapeutique optimale, la fraction déposée sur les étages 3 à 6, notés S3 à S6, et plus particulièrement sur S4 et S5, doit être maximisée.

La figure 1 montre que la composition pharmaceutique C4 selon l'invention, qui comprend 1% en masse d'éthanol, permet effectivement d'optimiser cette efficacité thérapeutique puisque la fraction de salbutamol déposée sur ces étages S3 à S6, et en particulier sur les étages S4 et S5, est très nettement supérieure aux fractions de salbutamol déposées sur ces mêmes étages à partir des compositions pharmaceutiques comparatives C1 à C3, ce phénomène étant plus marqué à partir de la composition pharmaceutique comparative C3. Par rapport aux données obtenues avec les compositions pharmaceutiques comparatives C1 et C2, on observe que la mise en œuvre de la composition pharmaceutique C4 conforme à l'invention permet un déplacement des particules fines de l'étage S3 vers l'étage S4 et surtout vers l'étage S5 qui sont inférieurs en taille de particules fines.

Ce constat est d'autant plus surprenant que :
- d'une part, les compositions pharmaceutiques comparatives C1 et C2 comprennent respectivement des proportions massiques de 0,25 % en masse et 0,3 % en masse qui s'inscrivent dans l'intervalle préféré de 0,2 % en masse à 1,0 % en masse de tensioactif, en l'espèce l'acide oléique, enseigné par le document [1], et
- d'autre part, la composition pharmaceutique comparative C3 ne comprenant ni tensioactif, ni éthanol, est décrite par le document [3] comme présentant de bonnes performances pharmaceutiques.

Les performances thérapeutiques de la composition pharmaceutique conforme à l'invention sont, en outre, corroborées par les données de fractions de fines particules rapportées dans le tableau 2 ci-dessous, tableau dans lequel les quantités d'acide oléique ou d'éthanol présentes dans les compositions pharmaceutiques C1, C2 et C4 ont été rapportées en proportion massique (% en masse).

**Tableau 2**

| Composition | Acide oléique (% en masse) | Ethanol (% en masse) | Fraction de fines particules (en %) |
|---|---|---|---|
| C1 | 0,25 | 0 | 15 |
| C2 | 0,3 | 0 | 16 |
| C3 | 0 | 0 | 13 |
| C4 | 0 | 1,00 | 21 |

### Exemple 2

Comme dans l'exemple 1, les aérosols-doseurs ont été préparés avec les mêmes références de composés, de cartouches en aluminium et de valves doseuses, selon un protocole opératoire identique.

Dans une première étape, une valve doseuse a été sertie sur chacune des cartouches avec un équipement adéquat.

Dans une deuxième étape, on a procédé au remplissage, au moyen d'un équipement pilote et en deux temps, de deux séries distinctes d'aérosols-doseurs par introduction via la valve doseuse :
- d'une suspension concentrée comprenant 30,125 mg de sulfate de salbutamol (diamètre médian de l'ordre de 5 µm), une quantité réduite de gaz propulseur R-152a et, le cas échéant, la proportion massique d'éthanol indiquée dans le tableau 3 ci-dessous, par rapport à la masse totale de composition pharmaceutique, puis
- d'une quantité suffisante de gaz propulseur R-152a pour atteindre une masse totale de composition pharmaceutique de 9,57 g.

**Tableau 3**

| Composition | C3' | C4' |
|---|---|---|
| Ethanol (% en masse) | 0 | 1,00 |

Trois séries de tests de mesure de distribution granulométrique aérodynamique (APSD) ont été réalisées à un débit de 30 L/min, en expulsant 5 doses de chacune des compositions pharmaceutiques C3' et C4' dans l'impacteur NGI, comme dans l'exemple 1 ci-dessus.

Une première série de tests de mesure de distribution granulométrique aérodynamique a été conduite sur les compositions pharmaceutiques C3' et C4' telles qu'obtenues à T0, c'est-à-dire au terme de la période de quarantaine mentionnée dans l'exemple 1.

Les résultats de cette première série de tests à T0 sont reportés sur la figure 2A.

Une deuxième série de tests de mesure de distribution granulométrique aérodynamique a été conduite sur ces mêmes compositions pharmaceutiques C3' et C4' telles qu'obtenues à T3M, c'est-à-dire au terme d'un stockage d'une durée de trois mois à compter de T0 des aérosols-doseurs comprenant lesdites compositions C3' et C4', ces aérosols-doseurs étant disposés, pendant ces trois mois, en position inversée (valve vers le bas) dans des conditions respectives de température et d'humidité relative de 40 °C et de 75 % qui sont conformes aux directives de l'International Council for Harmonisation for Pharmaceutical Quality (ICH Q1 Stability Guidelines).

Les résultats de cette deuxième série de tests à T3M sont reportés sur la figure 2B.

Une troisième série de tests de mesure de distribution granulométrique aérodynamique a été conduite sur ces mêmes compositions pharmaceutiques C3' et C4' telles qu'obtenues à T6M, c'est-à-dire au terme d'un stockage d'une durée de six mois à compter de T0 des aérosols-doseurs comprenant lesdites compositions C3' et C4', ces aérosols-doseurs étant disposés, pendant ces six mois, en position inversée et dans les conditions de température et d'humidité relative décrites dans le paragraphe précédent.

Les résultats de cette troisième série de tests à T6M sont reportés sur la figure 2C.

Les figures 3 et 4 regroupent les graphes des figures 2A à 2C obtenus avec la composition pharmaceutique C4' conforme à l'invention (figure 3) et avec la composition pharmaceutique comparative C3' (figure 4). Il est précisé que cette composition pharmaceutique comparative C3' est conforme à l'enseignement du document [3].

Les graphes des figures 2A à 2C, 3 et 4 représentent les fractions de salbutamol déposées, d'une part, au niveau de la gorge et de la bouche (T&M) et, d'autre part, sur chacun des huit étages de l'impacteur (notés S1 à S8).

Pour assurer une efficacité thérapeutique optimale, les fractions déposées sur les étages S3 à S6 doivent être maximisées et la fraction déposée au niveau T&M minimisée.

Les figures 2A, 2B et 2C montrent que la composition pharmaceutique C4' selon l'invention, qui comprend 1 % en masse d'éthanol, permet effectivement d'optimiser cette efficacité thérapeutique.

D'une part, en référence à la figure 2A, on constate que la somme des fractions de salbutamol déposées à T0 sur les étages S3 à S6 à partir de la composition pharmaceutique C4' est nettement supérieure à la somme des fractions de salbutamol déposées à T0 sur ces mêmes étages S3 à S6 à partir de la composition pharmaceutique comparative C3'. Cette observation est encore plus marquée si l'on se réfère aux graphes des figures 2B et 2C.

D'autre part, et toujours en référence à la figure 2A, on observe que la fraction de salbutamol déposée à T0 à partir de la composition pharmaceutique C4' selon l'invention au niveau T&M est de l'ordre de 35 % et donc bien inférieure à la fraction de salbutamol déposée à T0 à partir de la composition pharmaceutique comparative C3', qui est de l'ordre de 45%. Si l'on se réfère aux figures 2B et 2C, on observe que ce taux de 35 % est conservé à T3M et T6M avec la composition pharmaceutique C4' selon l'invention alors que celui-ci augmente pour atteindre des valeurs de l'ordre de 70 % avec la composition comparative C3'.

La figure 3 montre que la composition pharmaceutique C4' selon l'invention conserve cette efficacité thérapeutique optimisée dans le temps, même après six mois de stockage à 40 °C et 75 % d'humidité relative. En effet, les graphes de cette figure 3 sont pratiquement superposables, traduisant le fait que la somme des fractions de salbutamol déposées sur les étages S3 à S6 ainsi que la fraction de salbutamol déposée au niveau T&M sont similaires, voire identiques, à T0, T3M et T6M. En d'autres termes, la composition pharmaceutique C4' selon l'invention se caractérise par des performances d'aérosolisation stables dans le temps.

Au contraire, si l'on se réfère à la figure 4, on observe que l'efficacité thérapeutique est fortement dégradée pour la composition pharmaceutique comparative C3' pour le moins déjà après trois mois de stockage à 40 °C et 75 % d'humidité relative (T3M) et, *a fortiori,* à T6M.

### Bibliographie

[1] WO 2013/054137 A1
[2] WO 2014/170689 A1
[3] WO 2013/054135 A1
[4] US 2007/041911 A1
[5] US 2021/244688 A1

## Revendications

1. Composition pharmaceutique constituée des composés suivants :
(a) du sulfate de salbutamol en tant que principe actif,
(b) du 1,1-difluoroéthane (R-152a), et
(c) de l'éthanol.

2. Composition pharmaceutique selon la revendication 1, dans laquelle la proportion massique de principe actif (a) est comprise entre 0,05 % et 0,5 % en masse, avantageusement comprise entre 0,1 % et 0,4 % en masse et, de préférence, comprise entre 0,2 % et 0,35 % en masse par rapport à la masse totale de la composition pharmaceutique.

3. Composition pharmaceutique selon la revendication 1 ou 2, dans laquelle la proportion massique de 1,1-difluoroéthane (b) est comprise entre 89,5 % et 99,9 % en masse, notamment comprise entre 94,5 % et 99,9 % en masse, avantageusement comprise entre 96,6 % et 99,7 % en masse et, de préférence, comprise entre 97,65 % et 99,3 % en masse par rapport à la masse totale de la composition pharmaceutique.

4. Composition pharmaceutique selon l'une quelconque des revendications 1 à 3, dans laquelle la proportion massique d'éthanol (c) est comprise entre 0,05 % et 10 % en masse, notamment comprise entre 0,05 % et 5 % en masse, avantageusement comprise entre 0,2 % et 3 % en masse et, de préférence, comprise entre 0,5 % et 2 % en masse par rapport à la masse totale de la composition pharmaceutique.

5. Composition pharmaceutique selon l'une quelconque des revendications 1 à 4 pour une utilisation dans le traitement de troubles respiratoires, tels que l'asthme et les broncho-pneumopathies chroniques obstructives (BPCO).

6. Cartouche comprenant une composition pharmaceutique selon l'une quelconque des revendications 1 à 4.

7. Aérosol-doseur (MDI) muni d'une cartouche selon la revendication 6.

8. Utilisation de la composition pharmaceutique selon l'une quelconque des revendications 1 à 4 ou de la cartouche selon la revendication 6 dans un aérosol-doseur (MDI).

## Patentansprüche

1. Pharmazeutische Zusammensetzung, bestehend aus folgenden Verbindungen:
(a) Salbutamolsulfat als Wirkstoff,
(b) 1,1-Difluorethan (R-152a), und
(c) Ethanol.

2. Pharmazeutische Zusammensetzung nach Anspruch 1, wobei der Massenanteil des Wirkstoffs (a) zwischen 0,05 und 0,5 Massen-%, vorteilhafterweise zwischen 0,1 und 0,4 Massen-% und vorzugsweise zwischen 0,2 und 0,35 Massen-%, bezogen auf die Gesamtmasse der pharmazeutischen Zusammensetzung, liegt.

3. Pharmazeutische Zusammensetzung nach Anspruch 1 oder 2, wobei der Massenanteil von 1,1-Difluorethan (b) zwischen 89,5 und 99,9 Massen-%, insbesondere zwischen 94,5 und 99,9 Massen-%, vorteilhafterweise zwischen 96,6 und 99,7 Massen-% und vorzugsweise zwischen 97,65 und 99,3 Massen-%, bezogen auf die Gesamtmasse der pharmazeutischen Zusammensetzung, liegt.

4. Pharmazeutische Zusammensetzung nach einem der Ansprüche 1 bis 3, wobei der Massenanteil von Ethanol (c) zwischen 0,05 und 10 Massen-%, insbesondere zwischen 0,05 und 5 Massen-%, vorteilhafterweise zwischen 0,2 und 3 Massen-% und vorzugsweise zwischen 0,5 und 2 Massen-%, bezogen auf die Gesamtmasse der pharmazeutischen Zusammensetzung, liegt.

5. Pharmazeutische Zusammensetzung nach einem der Ansprüche 1 bis 4 zur Verwendung bei der Behandlung von Atemwegserkrankungen wie Asthma und chronisch obstruktiven Lungenerkrankungen (COPD).

6. Kartusche, umfassend eine pharmazeutische Zusammensetzung nach einem der Ansprüche 1 bis 4.

7. Aerosol-Dosierer (MDI), versehen mit einer Kartusche nach Anspruch 6.

8. Verwendung der pharmazeutischen Zusammensetzung nach einem der Ansprüche 1 bis 4 oder der Kartusche nach Anspruch 6 in einem Aerosol-Dosierer (MDI).

## Claims

1. Pharmaceutical composition consisting of the following compounds:
(a) salbutamol sulphate as active ingredient,
(b) 1,1-difluoroethane (R-152a), and
(c) ethanol.

2. Pharmaceutical composition according to claim 1, wherein the mass proportion of active ingredient (a) is between 0.05% and 0.5% by mass, advantageously between 0.1% and 0.4% by mass and, preferably, between 0.2% and 0.35% by mass relative to the total mass of the pharmaceutical composition.

3. Pharmaceutical composition according to claim 1 or 2, wherein the mass proportion of 1,1-difluoroethane (b) is between 89.5% and 99.9% by mass, in particular between 94.5% and 99.9% by mass, advantageously between 96.6% and 99.7% by mass and, preferably, between 97.65% and 99.3% by mass relative to the total mass of the pharmaceutical composition.

4. Pharmaceutical composition according to any one of claims 1 to 3, wherein the mass proportion of ethanol (c) is between 0.05% and 10% by mass, in particular between 0.05% and 5% by mass, advantageously between 0.2% and 3% by mass and, preferably, between 0.5% and 2% by mass relative to the total mass of the pharmaceutical composition.

5. Pharmaceutical composition according to any one of claims 1 to 4 for use in the treatment of respiratory disorders, such as asthma or chronic obstructive pulmonary disease (COPD).

6. Canister comprising a pharmaceutical composition according to any one of claims 1 to 4.

7. Metered-dose inhaler (MDI) provided with a canister according to claim 6.

8. Use of the pharmaceutical composition according to any one of claims 1 to 4 or of the canister according to claim 6 in a metered-dose inhaler (MDI).
